# EUROPÄISCHE PATENTSCHRIFT

(11) **EP 1 900 336 B1**
(45) Veröffentlichungstag und Bekanntmachung des Hinweises auf die Patenterteilung: **09.06.2010**
(21) Anmeldenummer: 06019346.3
(22) Anmeldetag: 15.09.2006
(51) Int. Cl.: A61B 19/00

(54) **Vorrichtung und Verfahren zum Messen geometrischer Eigenschaften medizintechnischer Behandlungsvorrichtungen, insbesondere zur automatischen Verifikation, Kalibrierung und Vermessung von Instrumenten für computerassistierte Chirurgie**
Device and method for measuring geometric properties of medical tools, in particular for automated verification, calibration and gauging of tools for computer assisted surgery
Appareil et méthode pour mesurer les caractéristiques géométriques d'un outil chirurgical, en particulier pour la vérification, la calibration et le mesurage automatique d'un outil pour la chirurgie assistée par l'ordinateur

(43) Veröffentlichungstag der Anmeldung: 19.03.2008
(73) Patentinhaber: BrainLAB AG, 85622 Feldkirchen (DE)
(72) Erfinder: Neubauer, Timo, 85586 Poing (DE); Plaßky, Norman, 99099 Erfurt (DE); Millahn, Manuel, 80799 München (DE)
(74) Vertreter: Engelhard, Maximilian

(56) Entgegenhaltungen:
- WO-A-97/29710
- WO-A2-01/54558
- WO-A2-97/29678
- US-A1- 2002 143 268
- US-A1- 2006 129 140

## Beschreibung

Die vorliegende Erfindung betrifft Vorrichtungen und Verfahren zum Messen geometrischer Eigenschaften medizintechnischer Behandlungsvorrichtungen insbesondere zur automatischen Verifikation, Kalibrierung und Vermessung von Instrumenten für den Einsatz in computer-assistierter Chirurgie. Dabei können insbesondere mit Hilfe von optischen Scanverfahren die Geometrie des Instruments und eines durch mindestens zwei Marker gebildetes Referenzsystems, welches permanent oder abnehmbar am Instrument befestigt sein kann, als dreidimensionales Modell ermittelt und anschließend vom Navigationssystem weiterverwendet werden. Sowohl ein Instrument ohne Referenzsystem als auch ein Instrument mit Referenzsystem sowie ein Referenzsystem (z.B. Markereinrichtung) für ein Instrument (z.B. ein mit einem Instrument verbindbares Referenzsystem) stellen Beispiele für eine medizintechnische Behandlungsvorrichtung dar.

Die Genauigkeit und damit Schnelligkeit des beispielhaften Scanprozesses wird maßgeblich über die bereits für dieses Instrument vorhandenen und zugänglichen Informationen gesteuert, wobei mindestens eine Verifikation, durchaus aber eine Kalibrierung und sogar eine vollständige Vermessung der Instrumenteneigenschaften durchgeführt werden kann oder sogar muss.

### Hintergrund

Bei computergestützten Operationen werden dem Operateur die Position und Orientierung von chirurgischen Instrumenten in Beziehung zu den anatomischen Strukturen des Patienten mit Hilfe eines Navigationssystems auf dessen Anzeigevorrichtung dargestellt. Notwendigerweise müssen für eine genaue Darstellung neben den anatomischen Strukturen auch die verwendeten Instrumente durch geeignete Referenzsysteme (z. B. Markereinrichtungen) verfolgbar sein, was üblicherweise durch aktive oder passive Markerstrukturen erreicht wird, die dem Navigationssystem ihre Position im Operationsfeld durch Emmision oder Reflexion von Infrarotstrahlung offenbaren.

Die anatomischen Strukturen werden zu Beginn durch Verwendung von navigierbaren Zeigegeräten in einem Registrierungsprozess mit den an ihnen befestigten Referenzsystemen in Beziehung gesetzt und sind dadurch im späteren Operationsprozess für das Navigationssystem räumlich verfolgbar.

Gleiches ist für die Instrumente und ihre Referenzsysteme (z. B. Markereinrichtungen) erforderlich, wobei hier die instrumententypischen Funktionselemente von besonderer Bedeutung sind. Dabei handelt es sich zum Beispiel um Werkzeugflächen (Schneiden, Spitzen etc.), die für die Bearbeitung von beispielsweise knöchernen Strukturen benutzt werden, wobei der Chirurg ein Navigationssystem einsetzt, um trotz möglicherweise eingeschränkter Sichtbarkeit genaue Informationen über deren Position und Orientierung zu erhalten. Die Darstellung der räumlichen Lage des Instruments und seiner Funktionselemente auf der Anzeigevorrichtung beruht auf einer Korrelation von hinterlegten Geometriedaten des Instruments und der räumlichen Daten, die vom Navigationssystem über das am Instrument angebrachte Referenzsystem ermittelbar sind. Sobald die hinterlegten Geometriedaten, die hauptsächlich die Funktionselemente in Bezug auf das Referenzsystem beschreiben, von der tatsächlichen Geometrie des Instruments abweichen (z.B. nach Beschädigung des Instruments), führt dies unweigerlich auch zu einer falschen Darstellung der Beziehung zwischen realem Instrument und zu behandelnder anatomischer Struktur, wenn weiterhin die in der Datenbank hinterlegten Informationen über die initiale Gestalt des Instruments verwendet werden.

### Stand der Technik

Gegenwärtig wird die Relation zwischen Funktionselement des Instruments und seinem Referenzsystem einmalig anhand von Fertigungsunterlagen festgelegt, wobei deren Einhaltung durch Vermessung nach dem Fertigungsprozess sichergestellt wird. Die Relation wird im Navigationssystem hinterlegt und für nachfolgende Operationen ausgelesen und verwendet. Diese sogenannten prä-kalibrierten Instrumente (z.B. Bohrführungen) werden mitunter prä-operativ mit navigierbaren Hilfsmitteln verifiziert, um die Maßhaltigkeit zu bestätigen. Bei unzureichender Maßhaltigkeit wird dies dem Benutzer angezeigt, welcher auf den Einsatz des Instruments verzichten sollte, wenn eine nachfolgend beschriebene Kalibrierung nicht möglich ist. Eine Anpassung der Modelldaten an das real existierende, eventuell abweichende Instrument, ist bei der Verifikation nicht möglich. Die erreichbare Genauigkeit der Verifikation hängt zwangsläufig von der Genauigkeit des Navigationssystems ab, da wiederum die jeweiligen Referenzsysteme zueinander in Relation gesetzt werden. Sichtbarkeitsprobleme und mitunter schlechte Handhabbarkeit beim gleichzeitigen Positionieren von Instrument und Hilfsmittel bewirken dabei tendenziell eine Verlängerung der Operationszeit.

Bei einem weiteren Verfahren wird vor dem Gebrauch des Instruments ein Kalibrierungsvorgang des Instruments durchgeführt. Dabei werden die für die Navigation erforderlichen aber noch offenen Werte funktionsrelevanter Parameter des Instruments (Länge, Durchmesser, ...) oder offene Werte für die Zuordung von Referenzsystem zu Funktionselement (z.B. Pfanneneinschläger mit variablen Pfannen) oder die Abweichungen des realen Instruments vom in der Datenbank hinterlegten Modell mittels eines navigierbaren Kalibrierungswerkzeugs bestimmt, vorrübergehend oder permanent im Navigationssystem gespeichert und bei Bedarf ausgelesen. Auch hier wirken sich die vorstehend beschriebenen Sichtbarkeits- und Handhabbarkeitsprobleme negativ auf die Operationszeit aus. Die Kalibrierung funktioniert gut für Instrumente mit einfachen Geometrien der Funktionselemente aber wiederum nur in den Grenzen der vom Navigationssystem erreichbaren Genauigkeit.

Die vollständige Vermessung insbesondere funktionsrelevanter Strukturen eines schwer zu kalibrierenden oder zu verifizierenden Instruments stellt ein drittes Verfahren dar, das gegenwärtig jedoch nicht zum Einsatz kommt, da weder die geeigneten Hilfsmittel zur Verfügung stehen, noch die erforderlichen Prozeduren in einem vertretbaren zeitlichen Rahmen vom OP-Personal durchgeführt werden könnten.

Gegenwärtig werden mittels einer Detektionseinrichtung (z. B. Kamera oder Ultraschalldetektor) Markereinrichtungen detektiert, die Beispiele für ein Referenzsystem darstellen. Derartige Detektionssysteme werden auch als Navigationssysteme bezeichnet, wie sie bei der IGS (image guided segery) verwendet werden. Bei den Markereinrichtungen handelt es sich typischerweise um drei Marker, die in fester und vorbestimmter relativer Lage zueinander angeordnet sind und insbesondere mechanisch verbunden sind. Die Marker können passive oder aktive Marker sein, wobei die passiven Marker Signale (z.B. Wellen und/oder Strahlung) reflektieren, die in ihre Richtung ausgesendet werden und die aktiven Marker selbst Ursprung der Signale (z.B. Strahlung und/oder Wellen) sind. Die von den (aktiven oder passiven) Markern ausgehenden Signale, bei denen es sich um Wellensignale oder Strahlungssignale handeln kann, werden von einer Detektionsvorrichtung (z. B. Kamera detektiert). Um eine Position der Markereinrichtung relativ zu der Detektionseinrichtung festzulegen, wird dabei vorzugsweise die Markereinrichtung bewegt, um der Detektionseinrichtung verschiedene Ansichten der Markereinrichtung zu bieten. Auf dieser Grundlage kann dann in bekannter Weise die relative Lage der Markereinrichtung relativ zu der Detektionseinrichtung, insbesondere in einem im Raum ruhenden Bezugssystem bestimmt werden. In diesem Zusammenhang wird auf die DE 196 39 615 A1 und die entsprechende US-Veröffentlichung 6,351,659 hingewiesen, die hiermit durch Bezugnahme in die vorliegende Offenbarung aufgenommen wurde.

Die durchgeführte Kalibrierung kann im Laufe der Zeit ihre Gültigkeit verlieren, insbesondere können die geometrischen Eigenschaften der medizintechnischen Behandlungsvorrichtung sich im Laufe der Zeit ändern. Dies kann durch Abnutzungserscheinungen oder wiederholte oder einmalige mechanische Belastung verursacht sein.

Aus US 2002/0143268 A1 ist eine Wandleranordnung bekannt, um die Stabilität eines Knochenimplantats zu detektieren. Vibrationen werden in dem Knochenimplantat erzeugt und die Antwort auf die Vibrationen detektiert. Ein EPROM kann verwendet werden, um Messdaten und die Zeit der Messung zu speichern.

WO 97/29710 offenbart einen Wandler zur Bestimmung der Position eines medizinischen Instruments.

Die EP-A-1 719 472 bezieht sich auf eine Vorrichtung und ein Verfahren zur Vermessung chirurgischer Instrumente, wobei RFIDs verwendet werden.

Als weiterer Stand der Technik wird auf die US-A-2006/0129140 und die WO-A-01/54558 verwiesen.

### Aufgabe der Erfindung

Aufgabe der Erfindung ist es das Risiko der Benutzung einer medizintechnischen Behandlungsvorrichtung zu reduzieren, für das Daten über geometrischen Eigenschaften vorliegen, die nicht mehr gültig sind.

Vorstehende Aufgabe wird durch die Gegenstände der unabhängigen Ansprüche gelöst. Vorteilhafte Ausführungsformen gehen aus den Unteransprüchen hervor.

Vorteilhaft wird ein Verfahren und Vorrichtungen zur Verifikation, Kalibrierung und Vermessung von Instrumenten für die computer-assistierte Chirurgie bereitgestellt, welche eine Erhöhung der Prozesssicherheit und mitunter eine Zeiteinsparung beim Einsatz von navigierten Instrumenten für chirurgische Prozeduren ermöglichen. Dabei soll die Anwendbarkeit nicht auf symmetrische oder anderweitig geometrisch einfache navigierbare Instrumente beschränkt sein, sondern es sollen auch Instrumente mit komplexen Geometrien für den Einsatz in computer-assistierter Chirurgie vorbereitet werden.

### Allgemeine Beschreibung

Die vorliegende Erfindung betrifft allgemein Messvorrichtungen und Verfahren zum Messen geometrischer Eigenschaften einer medizintechnischen Behandlungsvorrichtung, insbesondere eines Instruments mit Referenzsystem. Eine Verifikation, Kalibrierung und/oder _Vermessung der Medizintechnischen Behandlungsvorrichtung stellen ein Bespiel für ein Messen der geometrischen Eigenschaften der medizintechnischen Behandlungsvorrichtung dar. Eine geometrische Eigenschaft einer medizintechnischen Behandlungsvorrichtung ist erfindungsgemäß die relative Lage zwischen mindestens einem ersten Teil der medizintechnischen Behandlungsvorrichtung und mindestens einem zweiten Teil der medizintechnischen Behandlungsvorrichtung, wobei sich der zweite Teil vom ersten Teil unterscheidet. Beispielsweise kann der erste Teil ein Funktionselement des Instruments, z. B. eine Instrumentenspitze sein. Der zweite Teil kann ein Griff des Instruments sein oder eine am Instrument angebrachte Markereinrichtung. Auch kann der erste Teil eine erste Markerkugel einer Markereinrichtung und der zweite Teil eine zweite Markerkugel der Markereinrichtung sein.

Die Lage der oben genannten Teile wird bevorzugt durch die Position der Teile in einem vorbestimmen Bezugssystem bestimmt. Vorzugsweise wird als Bezugssystem ein Bezugssystem verwendet, in dem eine Detektionseinrichtung (z.B. Kamera) oder die Messvorrichtung (z.B. Scaneinheit) ruht. Die Positionen können zum Beispiel mit kartesischem Koordinaten oder Kugelkoordinaten beschrieben werden. Die relative Lage von einem Teil (z. B. erster Teil) zu einem anderen Teil (z. B. zweiter Teil) kann insbesondere durch Raumwinkel und/oder Abstände und/oder Koordinaten (in einem Bezugssystem) und/oder Vektoren beschrieben werden und wird vorzugsweise aus dem die Lage beschreibenden Positionen z. B. mittels eines Programms errechnet, das auf einem Computer läuft.

Der hierin verwendete Begriff "relative Lage" oder der Ausdruck "Lage eines Teils A relativ zu einem Teil B" umfasst also den Begriff der relativen Positionen zwischen den zwei Teilen.

Erfindungsgemäß soll die Zeit der Messung der geometrischen Eigenschaft erfasst werden und mittels eines RFID-Schreibgeräts in einen RFID-Transponder geschrieben werden, der sich z.B. in der medizintechnischen Behandlungsvorrichtung befindet. Dadurch kann, beim Lesen des RFID-Transponders bestimmt werden, wie lange die Messung zurück liegt und wie zuverlässig somit die Messdaten sind. Je länger der Messvorgang zurückliegt, desto wahrscheinlicher ist es, dass die gemessenen geometrischen Eigenschaften nicht mehr gültig sind. Desto dringender ist also eine neue Messung gefordert. Ein Beispiel für einen RFID-Transponder ist ein RFID-Etikett, ein RFID-Chip oder ein RFID-Tag. Die Abkürzung "RFID" steht für Radio Frequency Identification.

Die Zeiterfassungseinheit ist beispielsweise eine Uhr (insbesondere Funkuhr), die vorzugsweise mit der Messeinheit gekoppelt ist, insbesondere Signale von der Messeinheit empfängt. Die Zeiterfassungseinheit empfängt beispielsweise ein Signal, wenn die Messeinheit die Messung beginnt und/oder wenn die Messeinheit mit der Messung der geometrischen Eigenschaft oder der geometrischen Eigenschaften der medizinischen Behandlungsvorrichtung in Gang ist oder zu Ende ist. Die Zeiterfassungseinheit stellt dann die Zeit fest, wann dieses Signal empfangen wurde. Die Zeiterfassung kann von unterschiedlicher Genauigkeit sein. Beispielsweise kann die Tageszeit auf die Stunde genau, die Minute genau oder die Sekunde genau bestimmt werden. Beispielsweise kann auch nur das Datum, also Tag, Monat und Jahr oder auch nur die Woche und das Jahr oder der Monat und das Jahr bestimmt werden. Eine ungenaue Zeiterfassung ist insbesondere dann tolerierbar, wenn für die medizintechnische Behandlungsvorrichtung üblicherweise eine geometrische Eigenschaft erwartet wird, die über lange Zeit, beispielsweise Jahre typischerweise stabil ist.

Die vorliegende Erfindung ist insbesondere auch auf ein System aus der Messvorrichtung in Verbindung mit einer medizintechnischen Behandlungsvorrichtung gerichtet. Bei dem in der medizintechnischen Behandlungsvorrichtung verwendeten RFID-Transponder kann es sich insbesondere um einen handeln, der einen mehrfachbeschreibbaren Speicher umfasst. Auf diese Art und Weise ist es möglich, bei einer erneuten Vermessung der medizintechnischen Behandlungsvorrichtung die aktuelle Zeit der Messung in den RFID-Transponder einzuschreiben.

Die erfindungsgemäße Messvorrichtung ist so ausgebildet, dass das RFID-Schreibgerät Messdaten von der Messeinheit erhält. Beispielsweise ist das RFID-Schreibgerät signaltechnisch mit der Messeinheit verbunden oder mit einer Datenverarbeitungseinrichtung, die die gemessenen Daten verarbeitet. Vorzugsweise ist das RFID-Schreibgerät ausgebildet, zusätzlich zu der erfassten Zeit auch Messdaten der Messeinheit in den RFID-Transponder der medizintechnischen Behandlungsvorrichtung zu schreiben.

Erfindungsgemäß misst die Messeinheit zumindest die relative Lage zwischen einem ersten Teil der medizintechnischen Behandlungsvorrichtung und einem zweiten Teil der medizintechnischen Behandlungsvorrichtung. Dies bedeutet, dass auch eine umfassendere Messung, wie beispielsweise eine Kalibrierung, bei der die relative Lage vieler Teile der medizintechnischen Behandlungsvorrichtung zueinander bestimmt wird, umfasst ist. Die erfindungsgemäße Messeinheit kann als so genannter Pointer ausgebildet sein, d.h. ein Zeigegerät mit mindestens zwei Markerkugeln, das von einem Kamerasystem, insbesondere Navigationssystem während des Abtastvorgangs beobachtet wird. Beim Abtastvorgang werden Teile der medizintechnischen Behandlungsvorrichtung mittels des Pointers abgegriffen. Aus den Beobachtungssignalen wird die relative Lage zwischen den Teilen bestimmt.

Insbesondere ist die erfindungsgemäße Messeinheit als Scanneinheit ausgebildet, die die Geometrie eines Instruments einschließlich eines daran angebrachten Referenzsystems erfasst. Vorzugsweise umfasst die erfindungsgemäße Vorrichtung weiter eine Datenverarbeitungseinheit, die beispielsweise ausgebildet ist mittels der erfassten Geometrie des Instruments und des Referenzsystems ein dreidimensionales Modell des Instruments berechnet. Andere beispielhafte Funktionen der Datenverarbeitungseinheit sind unten beschrieben.

Gemäß einer bevorzugten Ausführungsform kann die Messvorrichtung eine Datenverarbeitungseinrichtung umfassen, die aus der erfassten Zeit, also der Zeit der Messung eine Soll-Zeit berechnet. Die Soll-Zeit kann als eine Verfallszeit der medizintechnischen Behandlungsvorrichtung aufgefasst werden. Nach dem Ablauf eines Zeitraums nach Messung der geometrischen Eigenschaften kann es sein, dass die geometrischen Eigenschaften nicht mehr gültig sind, insbesondere das Risiko erhöht ist, dass die geometrischen Eigenschaften nicht mehr gültig sind. Addiert man diesen Zeitraum zu der Zeit der Messung, so ergibt sich die vorgenannte Soll-Zeit. Die Soll-Zeit kann je nach Typ des vermessenen Instruments variieren. Vorzugweise umfasst deshalb die Datenverarbeitungseinrichtung eine Datenbank, in der in Abhängigkeit vom Instrumententyp verschiede Zeiträume (Soll-Zeiträume) abgespeichert sind, nach denen davon ausgegangen wird, dass die geometrischen Eigenschaften der medizintechnischen Behandlungsvorrichtung nicht mehr gütig sind. Die Zeit, die mittels des RFID-Schreibgeräts in den RFID-Transponder geschrieben wird, kann also die vorgenannte Messzeit und/oder die Soll-Zeit sein.

Der Typ der medizintechnischen Behandlungsvorrichtung kann beispielsweise von einem Benutzer eingegeben werden oder mittels der Messdaten basierend auf den typischen Geometriedaten der medizintechnischen Behandlungsvorrichtung oder basierend auf einem Modell bestimmt werden, dass anhand der Messdaten ermittelt wurde. Insbesondere kann es sich dabei um ein dreidimensionales Modell handeln, wie weiter unten in Zusammenhang mit der Vermessung beschreiben wird. Dadurch kann eine voll automatische Bestimmung des Typs des vermessenen Instruments durchgeführt werden. Insbesondere können hierzu verschiedene Modelle der medizintechnischen Behandlungsvorrichtungen (Instrumente und/oder Referenzsysteme) abgespeichert sein. Damit kann eine vollautomatische Bestimmung des Typs der medizintechnischen Behandlungsvorrichtung durchgeführt werden, wobei die Daten über den bestimmten Typ in Verbindung mit der bestimmten Zeit (Zeit der Messung und/oder Soll-Zeit) in den RFID-Transponder geschrieben werden. Somit kann auch der bestimmte Typ der medizintechnischen Behandlungsvorrichtung von dem RFID-Schreibgerät, das bevorzugt mit der Datenverarbeitungseinrichtung gekoppelt ist, in den RFID-Transponder geschrieben werden (z.B. zusätzlich zu den Zeit-Daten und Messdaten).

Weiter ist die Erfindung auf ein Navigationssystem gerichtet, das das Navigieren einer medizintechnischen Behandlungsvorrichtung erlaubt und auch die oben genannte Datenverarbeitungseinheit umfassen kann. Das Navigationssystem umfasst dabei ein RFID-Lesegerät, das die Zeit ausliest, die in dem RFID-Transponder der medizintechnischen Behandlungsvorrichtung gespeichert ist. Vorzugsweise ist das Navigationssystems so gestaltet, dass die ausgelesene Zeit mit der Soll-Zeit verglichen wird. Liegt die ausgelesene Zeit nach der Soll-Zeit so wird vorzugsweise von dem Navigationssystem eine Soll-Zeit-Überschreitungsoperation durchgeführt. Diese Soll-Zeit-Überschreitungsoperation umfasst beispielsweise das Abgeben eines Warnsignals, dass den Benutzer warnt, dass die geometrischen Eigenschaften der Behandlungsvorrichtung bereits seit längerer Zeit nicht mehr gemessen wurden, insbesondere sie nicht kalibriert wurde. Deshalb besteht ein erhöhtes Risiko, dass vom Navigationssystem verwendete Daten über die geometrischen Eigenschaften der medizintechnischen Behandlungsvorrichtung, insbesondere Kalibrierdaten nicht mehr gültig sind. Alternativ oder zusätzlich kann die Soll-Zeit- Überschreitungsoperation so gestaltet sein, dass ein Navigieren mit der medizintechnischen Behandlungsvorrichtung unterbunden wird. Dies ist insbesondere dann von Vorteil, wenn vom Hersteller der medizintechnischen Behandlungsvorrichtung eine Beibehaltung der geometrischen Eigenschaften nur für einen bestimmten Zeitraum garantiert wird. Vorzugsweise umfasst das Navigationssystem eine Datenverarbeitungseinheit, die beispielsweise die oben genannten Vergleichs- und/oder Soll-Zeit-Überschreitungsoperationen steuert und/oder durchführt.

Wird eine medizintechnische Behandlungsvorrichtung mit der erfindungsgemäßen Messvorrichtung erneut vermessen, insbesondere erneut kalibriert, so wird, wie oben ausgeführt, vorzugsweise der RFID-Transponder der medizintechnischen Behandlungsvorrichtung ausgelesen. Vorzugsweise werden sowohl die erfasste Zeit als auch die früher gemessenen geometrischen Eigenschaften ausgelesen. Vorzugsweise vergleicht die Datenverarbeitungseinheit, die aus dem RFID-Transponder ausgelesenen geometrischen Daten mit den neu gemessenen Daten. Unterscheiden sich diese Daten zumindest in einem vorbestimmten Umfang, so wird vorzugsweise eine Soll-Zeit, bei der eine erneute Messung durchgeführt werden soll festgelegt, die nach einem Zeitraum liegt, der kürzer ist als der Zeitraum zwischen der letzten Messung und der aktuellen Messung. Unterscheiden sich die neu gemessenen geometrischen Eigenschaften nicht von dem im RFID-Transponder gespeicherten geometrischen Eigenschaften, so wird eine Soll-Zeit vorzugsweise gesetzt, die nach einem Zeitraum liegt, der gleich dem Zeitraum zwischen der letzten Messung und der aktuellen Messung ist oder länger ist.

Vorzugsweise werden bei einem erfindungsgemäßen Navigationssystem, die im RFID-Transponder gespeicherten Daten über die geometrischen Eigenschaften verwendet, um die medizintechnische Behandlungsvorrichtung zu navigieren, insbesondere dreidimensional darzustellen.

Der erfindungsgemäße RFID-Transponder kann sich im Inneren der medizintechnischen Behandlungsvorrichtung befinden oder daran angebracht sein. Beispielsweise kann im Inneren ein RFID-Chip eingebaut sein oder auf die Oberfläche der medizintechnischen Behandlungsvorrichtung kann ein RFID-Etikett aufgeklebt sein.

Das Referenzsystem, das insbesondere als Markereinrichtung ausgebildet ist, kann ebenfalls den RFID-Transponder alternativ oder zusätzlich zu einem RFID-Transponder des Instruments umfassen. Insbesondere kann ein RFID-Transponder der Markereinrichtung charakteristische Daten der Markereinrichtung insbesondere die relative Lage der Marker (z. B. Markerkugeln) zueinander enthalten, die charakteristisch für eine Markereinrichtung sind.

Vorteilhaft dienen die Verifikation, Kalibrierung und Vermessung mittels der Messvorrichtung dazu, dass dem Navigationssystem möglichst vor Beginn der Operation korrekte Instrumentendaten über die Geometrie von den Funktionselementen und dem Referenzsystem (z. B. Markereinrichtungen) sowie deren Relation zueinander bereitgestellt und im Speicher des Navigationssystems temporär oder permanent hinterlegt werden, wobei auch eine intra-operative Anwendung der Verifikation, Kalibrierung und Vermessung nicht ausgeschlossen werden soll, um zum Beispiel zwischenzeitlich deformierte Instrumente nach der Durchführung der beschriebenen Verfahren erneut benutzen zu können. Ein erfindungsgemäßes Navigationssystem ist vorzugsweise ausgebildet diese Daten direkt aus dem RFID-Transponder der medizintechnischen Behandlungsvorrichtung auszulesen.

Die Instrumentendaten können Angaben über die Lage der Funktionselemente des Instruments (z. B. Instrumentenspitze) in Bezug auf ein fest angebrachtes oder abnehmbares, aus Markern (aktive oder passive Technologie) gebildetes Referenzsystem beinhalten. Die Daten können insbesondere auch angeben, wie die Funktionselemente, wie z.B. die Spitzen des Instruments, geformt sind.

Bei den erfindungsgemäßen Verfahren wird vorzugsweise mittels einer vorzugsweise berührungslos arbeitenden Scanvorrichtung oder Abtastvorrichtung die Geometrie des Instruments inklusive einem befestigten oder befestigbaren, aus mindestens zwei Markern gebildeten Referenzsystems ermittelt und mit bereits hinterlegten, dem Instrument oder seiner Instrumentengruppe eindeutig zuordenbaren Werten verglichen bzw. bei Vermessung neu abgelegt. Insbesondere können das Referenzsystem oder die Marker dauerhaft an dem Instrument angeordnet sein oder können zeitweise, z.B. während des Scanvorganges, angebracht werden. Die durch das Scannen gewonnenen Oberflächendaten des Instruments werden dabei mittels einer Datenverarbeitungseinheit oder Recheneinheit in ein dreidimensionales Modell des Instruments umgewandelt, anhand dessen die instrumententypischen Kennwerte berechnet werden können. Das dreidimensionale Modell des Instruments kann dabei alle zur Navigation notwendigen Informationen über die Geometrie der Funktionselemente des Instruments und seines Referenzsystems sowie deren Beziehung zueinander beinhalten. Abschließend können die geometrischen Informationen über das Instrument dem Navigationssystem übergeben werden, um dem Operateur die Verwendung des Instruments in der computer-assistierten Chirurgie zu ermöglichen.

### Messvorrichtung und Verfahren

Die erfindungsgemäßen Vorrichtungen und Verfahren werden nachfolgend beschrieben.

### Messeinheit

Die Scanvorrichtung ist ein Beispiel für eine Messeinheit, die entweder im Gehäuse des Navigationssystems integriert oder in einem separaten Gehäuse untergebracht sein kann. Die Scanvorrichtung verwendet bekannte 3D-Scantechniken, wie z.B. Lichtschnittverfahren, Objektrasterverfahren, welche eine gerasterte Instrumentenoberfläche erfordern, oder Gitterprojektionsverfahren, bei welchen telezentrisches Gitter auf die Instrumentenoberfläche projiziert werden. Die in der Scanvorrichtung befindliche Scaneinheit arbeitet vorzugsweise optisch, wie mittels eines Lasers, wobei die Oberfläche des Instruments inklusive dauerhaft oder abnehmbar befestigtem Referenzsystem abgetastet wird. Dabei wird das zu scannende Instrument in einem in der Scanvorrichtung enthaltenen Instrumentenhalter befestigt, wobei während das Scanvorgangs dieser Instrumentenhalter relativ zur Scaneinheit bewegt oder gedreht werden kann, um das Instrument der Scaneinheit von allen Seiten zugänglich und somit eine vollständige Digitalisierung der Oberfläche möglich zu machen. Alternativ kann sich auch die Scaneinheit relativ zu einem fixen Instrumentenhalter bewegen oder drehen, wobei in beiden Fällen die Bewegung oder Drehbewegungen über geeignete, in der Scanvorrichtung integrierte Sensorik erfasst oder gesteuert werden kann, um die Winkelstellungen eindeutig mit den Scanergebnissen korrelieren zu können, wodurch ein realitätsgetreues 3D-Model berechenbar wird.

Zusätzlich kann während des Scanvorgangs mittels einer Infrarot-Kameraeinheit, die separat vorhanden oder in der Scanvorrichtung enthalten sein kann, aber deren Lage in Bezug auf die Scaneinheit fest und bekannt oder ermittelbar ist, ein Emmisions- bzw. Reflexionsmodell der das Referenzsystem bildenden aktiven bzw. passiven Marker bestimmt werden. Die dadurch ermittelten Informationen über die Strahlungseigenschaften der Marker in Abhängigkeit vom Betrachtungswinkel und der sich daraus ergebenden, vom Navigationssystem erfassbaren, optischen Gestalt als räumliche Lage des Referenzsystems, können mit den Ergebnissen aus der Bestimmung seiner geometrischen Gestalt (mittels Scanprozess) korreliert werden. Damit lassen sich während des operativen Einsatzes Betrachtungsfehler des Navigationssystems, die aus sich änderndem Sichtbarkeitsverhalten der Marker in bestimmmten Lagen, z.B. durch partielle Beschädigung der Marker, resultieren können, kompensieren, was eine Erhöhung der Genauigkeit der Darstellung der Lage des Instruments in Bezug auf die zu behandelnde anatomische Struktur auf der Anzeigevorrichtung des Navigationssystems bewirkt. Mit anderen Worten können beim späteren Navigationsvorgang die auf Grund z.B. beschädigter Marker auftretenden Fehlinterpretationen der räumlichen Lage des Referenzsystems durch die Kameraeinheit des Navigationssystems kompensiert und die realitätsgetreue Lage des Instruments insbesondere des Funktionselements oder der Funktionselemente in Bezug auf die anatomische Struktur berechnet und dargestellt werden.

### Datenverarbeitungseinheit

Die erfindungsgemäße Messvorrichtung umfasst weiter vorzugsweise eine Recheneinheit oder Datenverarbeitungseinheit. Die von der Scaneinheit erfassten Daten über die Geometrie des navigierbaren Instruments können z.B. an die Datenverarbeitungseinheit gesendet und in dieser weiterverarbeitet werden, indem z.B. die Datenverarbeitungseinheit aus den Ergebnissen des Oberflächenscans ein dreidimensionales Modell des Instruments inklusive des Referenzsystems berechnet. Vorzugsweise kann die Datenverarbeitungseinheit die erfassten Daten oder das erstellte dreidimensionale Modell so auswerten, dass die Geometrie der Funktionselemente des Instruments (z.B. Spitzen) und deren Lage zum Referenzsystem des Instruments ermittelt werden können.

### Datenbank

Weiter kann die erfindungsgemäße Vorrichtung eine Datenbank umfassen, welche mit der Datenverarbeitungseinheit verbunden sein kann, so dass z.B. in die Datenverarbeitungseinheit eingegebene Daten oder an die Datenverarbeitungseinheit gesendete Daten in der Datenbank gespeichert werden können. So können beispielsweise Informationen über die Geometrie der Funktionseinheiten und des Referenzsystems des Instruments sowie deren Lage zueinander für verschiedene Instrumente aus der Datenverarbeitungseinheit in die Datenbank eingespeichert werden, genauso wie Informationen über die Qualität der Marker oder die Eigenschaften des Emmisions- bzw. Reflexionsmodels des instrumentenspezifischen Referenzsystems. Auch können geometrische Eigenschaften verschiedener Typen von Instrumenten und/oder Referenzsystemen gespeichert werden, so dass aus den gemessenen geometrischen Eigenschaften der Typ bestimmt werden kann.

### Anzeigevorrichtung

Auch kann eine Anzeigevorrichtung, insbesondere ein Bildschirm, der mit der Datenverarbeitungseinheit und der Benutzerschnittstelleneinheit drahtgebunden oder drahtlos (z.B. WLAN oder Bluetooth) kommuniziert, in der erfindungsgemäßen Messvorrichtung vorhanden sein. Die Darstellung der ermittelten Informationen über die Geometrie der Funktionselemente des Instruments und seines Referenzsystems sowie deren Lage zueinander kann in Form von Werten oder grafisch erfolgen. Vorzugsweise wird auf der Anzeigevorrichtung das aus der Datenbank ausgelesene oder durch Verarbeitung der Scannergebnisse ermittelte dreidimensionale Modell des Instruments angezeigt, wobei das Modells zumindest nahezu exakt auf der Anzeigevorrichtung dargestellt wird. Die realitätsnahe Darstellung des dreidimensionalen Modells auf der Anzeigevorrichtung gestattet es dem Benutzer unter Nutzung einer Benutzerschnittstelleneinheit, die vorzugsweise auf der Anzeigevorrichtung angeordnet ist, wie z.B. bei einem Touchscreen, notwendige Befehle für die korrekte Ausführung des erfindungsgemäßen Verfahrens an die Datenverarbeitungseinheit zu übertragen. Diese Befehle können die Auswahl des richtigen Modells aus einer Mehrzahl an ähnlichen Modellvarianten steuern, falls das Instrument nach dem Einbringen in die Scanvorrichtung nicht korrekt oder gar nicht erkannt wird. Weiterhin kann die, nach einem Grobscan mögliche grafische Darstellung von der Scanvorrichtung bis dahin unbekannten Instrumenten dafür genutzt werden, die für das erfolgreiche Durchführen der Instrumentenaufbereitung durch Vermessung erforderlichen Bereiche des Instruments, im besonderen die Funktionselemente und das Referenzsystem, interaktiv zu selektieren. Ebenso lassen sich für neue Instrumente Bezeichnungen eingeben, die das spätere Auffinden in der Datenbank erleichtern.

### Navigationssystemanbindung

Auch kann das Navigationssystem drahtlos oder drahtgebunden mit der Messvorrichtung oder der Datenverarbeitungseinheit verbunden sein, so dass die nach den erfindungsgemäßen Verfahren ermittelten Informationen über die Geometrie der Funktionselemente und des Referenzsystems des Instruments sowie deren Lage zueinander, und auch die Qualität der aktiven bzw. passiven Marker und deren daraus resultierendes Emmisions- bzw. Reflexionsmodell an das Navigationssystem, insbesondere an dessen Datenbank übertragen werden können. Mittels der Informationen kann das Navigationssystem den Operateur durch Bereitstellung der zuvor oder gerade ermittelten oder aktuellsten instrumentenspezifischen Daten bei der Durchführung der computer-assistierten Chirurgie unterstützen. Wie oben ausgeführt, erfasst das erfindungsgemäße Navigationssystem bevorzugt die Daten oder einen Teil der Daten von dem RFID-Transponder der medizintechnischen Behandlungsvorrichtung, so dass eine datentechnische Verbindung zwischen Navigationssystem und Messvorrichtung nicht oder nicht immer notwendig ist.

### Verfahren zur automatischen Verifikation, Kalibrierung und Vermessung von navigierbaren chirurgischen Instrumenten

Das erfindungsgemäße Verfahren von navigierbaren chirurgischen Instrumenten und die zugeordnete Zeiterfassung mit seinen unterschiedlichen Anforderungen und Bearbeitungsabläufen wird nachfolgend beschrieben.

Bei der Verifikation sind vorteilhaft die vollständigen geometrischen Daten des Instruments bereits in einer Datenbank des Navigationssystems hinterlegt, was bei sogenannten prä-kalibrierten Instrumenten die Regel ist. Die Durchführung einer Verifikation dieser Instrumente entspricht einer Überprüfung, ob die hinterlegten Daten mit der tatsächlichen Geometrie, insbesondere der Form der Funktionselemente und ihrer Relation zu dem Referenzsystem, übereinstimmen. Ein Unterlassen der Verifikation kann dazu führen, dass die Operation mit einem, nicht unbedingt offensichtlich beschädigten Instrument durchgeführt wird und somit auch die vorhandene Lage des Instruments zur anatomischen Struktur vom Navigationssystem nicht korrekt dargestellt wird.

Das Navigationssystem kann bevorzugt die verwendeten Instrumente anhand der charakteristischen und unterscheidbaren räumlichen Anordnung der das Referenzsystem bildenden Marker identifizieren, was nach einem erfolgreichen Erkennen das Auslesen der instrumentenspezifischen Daten aus der Datenbank zur Weiterverwendung während der Navigation ermöglicht.

Da sich die Scanvorrichtung zur Durchführung der Verifikation im Blickfeld des Kamerasystems befinden kann aber nicht muss und dadurch eine Identifizierung mit Hilfe des Navigationssystems nicht unbedingt sichergestellt werden kann, ist es mitunter sinnvoll auch andere ldentifizierungsprozeduren vorzuschlagen, die einen gezielten Abruf der Instrumentendaten durch die Scanvorrichtung ermöglichen. Die Erfüllung der erfindungsgemäßen Aufgabe einer automatischen Verifikation von navigierbaren chirurgischen Instrumenten kann eine Instrumentenidentifizierung mittels Strichcode (Barcode), NFC (Near Field Communication) oder besonders bevorzugt RFID (Radio Frequency Identification) beinhalten, wobei die Informationsträger automatisch beim Einsetzen des Instruments in die Scanvorrichtung von der Scanvorrichtung oder, falls das Instrument im Datenerfassungsberich des Navigationssystems ist von dem Navigationssystem erfasst werden können. Diese mobilen Informationsspeicher enthalten bevorzugt die geometrischen Daten des Instruments und zugeordnet die Zeit der Erfassung der geometrischen Daten. Auch kann der mobile Speicher die notwendigen Instrumenteninformationen bereitstellen, anhand derer ein schnelles Auffinden der instrumentenspezifischen Daten sowohl, als auch der zugeordneten Zeitdaten in der Datenbank des Navigationssystems möglich ist. Weiterhin ist die Auswahl des zu verfizierenden Instrumentes durch den Benutzer über eine geeignete, insbesondere grafische Schnittstelle denkbar, die entweder mit Hilfe der Anzeigevorrichtung des Navigationssystems oder über eine der Messvorrichtung (z.B. Scanvorrichtung) zugehörige Anzeigevorrichtung dargestellt werden kann. Ebenso können der Messvorrichtung (z.B. Scanvorrichtung) die, zum Auffinden der in der Datenbank abgelegten Geometriedaten, erforderlichen Informationen über das Instrument durch eine manualle Eingabe, z.B. mittels eines Instrumentenidentifizierungscodes, übergeben werden. Eine Verifikation ist ebenfalls möglich indem nach Einsetzen des Instruments in die Scanvorrichtung ein Initialscan oder Grobscan mit reduzierter Detailtreue durchgeführt wird, der in kürzester Zeit ein Modell des Instruments mit geringer oder verringerter Auflösung oder eine Grobstruktur des Instruments ermittelt, welches bzw. welche die Suche des Instruments durch Abgleich mit in der Datenbank hinterlegten Instrumentenmodellen gestattet. Auch hier werden nach erfolgreicher Suche die in der Datenbank des Navigationssystem gespeicherten vollständigen Instrumentendaten ausgelesen und bei der anschließenden Verifikation verwendet. Auch kann nach dem Grobscan des Instruments ein Feinscan oder ein weiterer oder zweiter Scanvorgang des Instruments oder Teilen des Instruments mit höherer oder erhöhter Detailtreue durchgeführt werden, woraus ein Modell mit hoher oder höherer Auflösung oder eine detaillierte oder genauere Struktur ermittelt werden kann.

Für alle beschriebenen Varianten der Verifikation ist es vorteilhaft, dass nach der Instrumentenidentifizierung mindestens die Funktionselemente und das Referenzsystem des Instruments, insbesondere deren Form und Lage zueinander, mit hoher Detailtreue gescannt werden, um ausreichend genaue Daten für den Vergleich der in der Datenbank hinterlegten Geometrie mit der tatsächlichen Instrumentengeometrie zu erhalten. Dabei kann erfindungsgemäß eine Erkennungslogik genutzt werden, die selbständig die charakteristischen Instrumenten-elemente identifiziert und damit eine Festlegung des Instrumentenbezugsystems zum Beispiel anhand der das Referenzsystem bildenden Marker festlegen und die Lage darauf bezogener Funktionselemente ermitteln kann. Die Identifizierung des Referenzsystems ist bei der Verwendung von z.B. passiven kugelförmigen Markern durch deren leicht zu erkennende Form, welche sonst eher selten bei chirurgischen Instrumenten vorkommt, sichergestellt. Instrumente mit aktiven Marker, die im Falle einer gewünschten Erfassung des Emmisionsmodells während des Scanvorganges mit einer Energiequelle verbunden sein können, können zum leichteren Auffinden der geometrisch kleinen Dioden (durch die Erkennungslogik nach erfolgtem Grobscan) in deren Umgebung ebenfalls mit geometrisch größeren charakteristischen Formen ausgebildet sein. Bei Nutzen einer externen, durch mit Kabeln verbundenen Energiequelle ist vorzugsweise die Aufnahemvorrichtung ortsfest auszuführen während sich die Scaneinheit relativ zu dieser bewegt.
Bei positivem Ergebnis der Verifikation, was einem Gleichen des Instruments mit dem hinterlegten Modell innerhalb einer vorgegebenen Toleranz entspricht, kann dies dem Navigationssystem über eine geeignete Datenverbindung (drahtgebunden, drahtlos) übermittelt werden und das Instrument kann für die nachfolgende Anwendung freigeschaltet werden.

### Kalibrierung

Die automatische Kalibrierung von navigierbaren chirurgischen Instrumenten ist in seinen Vorraussetzungen und dem Verfahrensablauf insbesondere identisch mit der vorstehend beschriebenen Verifikation. Während bei der Verifikation nur eine Aussage über die Gleichheit von Modell und realem Instrument getroffen wird, was bei Abweichungen außerhalb oder größer als der zulässigen Toleranz zum Ausschluss des Instruments vom anschließenden Navigationseinsatz bewirkt, werden bei der Kalibrierung die in der Datenbank des Navigationssystems abgespeicherten geometrischen Informationen des Instruments dahingehend korrigiert, dass das Instrument in den außerhalb der zulässigen Toleranzen liegenden Bereichen gescannt wird und die Ergebnisse als aktualisierte Modelldaten in die Datenbank des Navigationssystems übertragen werden.

### Vermessung

Bei der Vermessung ist es nicht erforderlich, dass geometrische Daten des Instruments bereits in einer Datenbank des Navigationssystems hinterlegt sind oder dass das Instrument dem Navigationssystem oder der Scanvorrichtung bekannt ist. Die erfindungsgemäße automatische Vermessung beginnt wie bei dem vorstehend beschriebenen Verfahren damit, dass das zu vermessende, mit passiven oder aktiven Markern ausgerüstete Instrument in dem Instrumentenhalter der Scanvorrichtung befestigt wird. Anschließend kann ein Scannen des Instruments zur Ermittlung eines dreidimensionalen Modells des Instruments oder ein Initialscan oder Grobscan mit reduzierter Detailtreue oder geringer Auflösung durchgeführt werden, wonach mit Hilfe der Datenverarbeitungseinheit ein grobes dreidimensionales Modell des Instruments oder ein Instrumentenmodell mit geringer Auflösung berechnet werden kann, das dann dem Benutzer auf der Anzeigevorrichtung dargestellt werden kann. Durch Nutzung der erfindungsgemäßen Erkennungslogik zum Auffinden charakteristischer, z.B. das Referenzsystem bildenden geometrischen Formen kann die Scanvorrichtung, nach erster Auswertung der Geometriedaten des Initialscans, durch farbliche oder anderweitige Kenntlichmachung dem Benutzer Vorschläge zur Festlegung der das Referenzsystem bildenden Marker und der Funktionselemente des Instruments unterbreiten. Der Benutzer kann die Vorschläge bestätigen oder Verbesserungen mit Hilfe der Benutzerschnittstelleneinheit, die vorzugsweise als Touchscreen ausgeführt ist, durchführen. Die Datenverarbeitungseinheit kann diese Angaben zur Festlegung des Scanbereiches für den nachfolgenden oder zweiten Scanvorgang nutzen, der insbesondere in den definierten Bereichen der Funktionselemente des Instruments und seines Referenzsystems mit hoher oder erhöhter Genauigkeit oder Auflösung durchgeführt werden kann. Die gewonnen Oberflächeninformationen können anschließend drahtgebunden oder drahtlos an die Datenverarbeitungseinheit übertragen werden, welche die exakten geometrischen Werte für die Lage der das Referenzsystem bildenden Marker und deren Abstand zu den Funktionselementen (z.B. Spitzen) des Instruments berechnen kann. Die dadurch bestimmten instrumentenspezifischen Kennwerte können wiederum drahtlos oder drahtgebunden an die Datenbank des Navigationssystems übertragen werden, welches bei Erkennung der charakteristischen, durch mindestens zwei aktive oder passive Marker gebildeten Referenzgeometrie die zugehörigen Informationen über die Lage des Funktionselements aus der Datenbank auslesen kann. Für eine erfindungsgemäß spätere Verifikation oder Kalibrierung des Instruments ist es denkbar die Instrumentengeometrie in der Datenbank unter einem vom Benutzer über die Benutzerschnittstelleneinheit eingegebenen Namen zu speichern, um das erforderliche Auffinden zu erleichtern. Besonders bevorzugt wird ein mobiler, am Instrument befestigter Datenspeicher der z.B. RFID (Radio Frequency Identification) Technologie verwendet, benutzt. Damit können die für spätere, insbesondere auf anderen Messvorrichtungen durchzuführenden Verifikationen oder Kalibrierungen notwendigen geometrischen Informationen am Instrument gespeichert und/oder ausgelesen werden. Erfindungsgemäß wird die Zeit der Messung gespeichert und/oder ausgelesen werden.

### Ergebnis der Messung, insbesondere der automatischen Verifikation, Kalibrierung und Vermessung von Instrumenten für computer-assistierte Chirurgie

Nach der Durchführung der erfindungsgemäßen Messung, insbesondere automatischen Verifikation, Kalibrierung und/oder Vermessung von navigierbaren chirurgischen Instrumenten in Verbindung mit der Zeiterfassung mit der die Zeit der Messung erfasst wird, kann mit Hilfe des Navigationssystems die Raumposition des mindestens aus zwei aktiven oder passiven Markern gebildeten Referenzsystems des Instruments bestimmt, aus der charakteristischen Lage der Marker zueinander das Instrument identifiziert, aus der Datenbank des Navigationssystems ein vollständiges Modell des Instruments (inklusive Referenzsystem und Funktionselemente) geladen, und/oder seine Lage in Bezug auf die zu behandelnde anatomische Struktur dem Operateur auf der Anzeigevorrichtung des Navigationssystems hochgenau dargestellt werden.

Die Erfindung bezieht sich weiterhin auf ein Computerprogramm, welches, wenn es in eine Meßvorrichtung gemäß der Erfindung geladen wird oder auf einer solchen Meßvorrichtung läuft, die wie oben beschriebenen Verfahren durchführt.

### Bildbeschreibung

Die Erfindung wird nachfolgend anhand bevorzugter Ausführungsbeispiele beschrieben. Es zeigen:
- Figur 1: eine erfindungsgemäße Vorrichtung zur automatischen Verifikation, Kalibrierung und Vermessung eines Instruments 700 mit separater Anzeigevorrichtung 300, sowie separater Datenverarbeitungseinheit 400;
- Figur 2: eine weitere Ausführungsform der erfindungsgemäßen Vorrichtung zur automatischen Verifikation, Kalibrierung und Vermessung eines Instruments 700 mit einer an einem Navigationssystem 600 angebrachten Anzeigevorrichtung 300 und einer in das Navigationssystem 600 integrierten Datenverarbeitungseinheit 400;
- Figur 3: eine erfindungsgemäße Scanvorrichtung mit dem aufzubereitenden Instrument 700; und
- Figur 4: eine erfindungsgemäße Anzeigevorrichtung 300.
- Figur 5: eine erfindungsgemäße Ausführungsform eines Navigationssystems

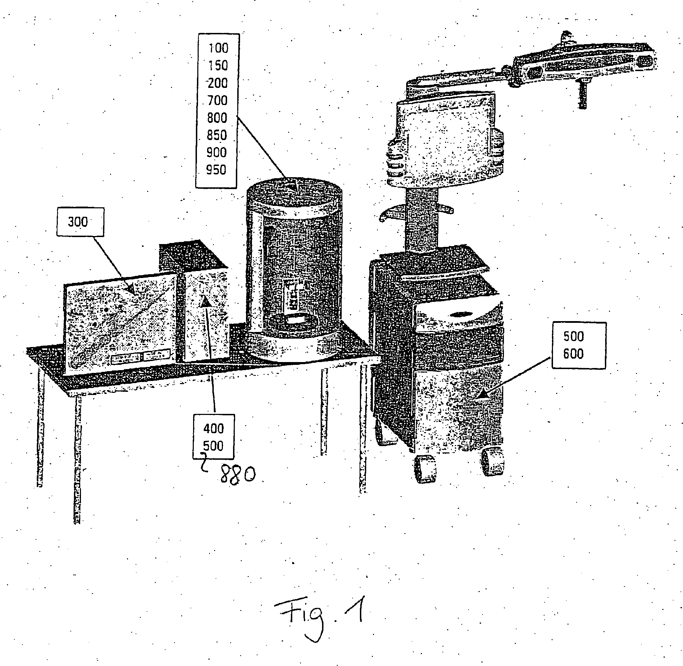

Figur 1 zeigt eine erste Ausführungsform der vorliegenden Erfindung, wobei eine Scanvorrichtung 100 und eine Instrumentenhalteeinheit 200 in einem zylinderförmigen Gehäuse angeordnet sind. Auch kann in dem Gehäuse eine Infrarot-Kameraeinheit 150 angebracht sein, um den Zustand oder die Form oder die Qualität von aktiven oder passiven Markern überprüfen zu können, indem zum Beispiel Infrarot-Strahlung auf die Marker gestrahlt wird und die reflektierte Infrarot-Strahlung von der Infrarot-Kameraeinheit 150 erfasst wird oder indem von den Markern emittierte Infrarot-Strahlung von der Infrarot-Kameraeinheit 150 erfasst wird. Das Instrument 700 ist in der vorliegenden Ausführungsform innerhalb des Gehäuses in der Instrumentenhalteeinheit 200 vorzugsweise fest oder unbeweglich positioniert, wobei auf dem Instrument 700 ein Referenzsystem 800 angebracht ist und das Instrument 700 als Funktionselement eine Instrumentenspitze 900 aufweist. Ein RFID-Tag 950 befindet sich im Inneren des Instruments 700 und/oder des Referenzsystems 800.

Die Scanvorrichtung 100 tastet das Instrument vorzugsweise optisch, zum Beispiel mittels eines Lasers oder taktil ab, wobei das Gehäuse der Scanvorrichtung 100 wie in der vorliegenden Ausführungsform offen aber auch geschlossen sein kann. Auch kann die Scanvorrichtung 100 oder der Laser der Scanvorrichtung 100 eine Drehbewegung um das Instrument ausführen oder die Scanvorrichtung 100 oder das Gehäuse der Scanvorrichtung 100 kann so ausgebildet sein, dass eine Drehung der Instrumentenhalteeinheit 200 oder des Instruments 700 ausgeführt werden kann und die Form des Instruments 700 von allen Seiten erfasst wird. Das Gehäuse der Scanvorrichtung 100 besitzt insbesondere eine Größe, auf Grund derer gängige Operationsinstrumente in dem Gehäuse der Scanvorrichtung 100 positioniert und vorzugsweise vollständig abgetastet werden können. Die Infrarot-Kameraeinheit 150, die in der vorliegenden Ausführungsform in oder an dem Gehäuse der Scanvorrichtung 100 angebracht ist, kann die Qualität, insbesondere das gleichmäßige Reflexionsverhalten der Marker untersuchen, indem zum Beispiel die Marker aus verschiedenen Winkeln von der Infrarot-Kameraeinheit 150 bestrahlt werden und aus der Reflexionscharakteristik der Marker ermittelt wird, welchen Zustand oder welche Qualität die Marker aufweisen. So kann zum Beispiel ein gleichmäßiges Reflexionsverhalten der Marker auf einen guten Zustand, oder intakten Zustand oder eine hohe Qualität der Marker hinweisen, während ein ungleichmäßiges Reflexionsverhalten oder eine ungleichmäßige Reflexionscharakteristik, die mit der Infrarot-Kameraeinheit 150 erfasst wurde, auf einen beschädigten Zustand oder eine geringe Qualität der Marker hinweisen kann.

Aus der ermittelten Qualität oder dem Zustand oder der Form der Marker oder des Referenzsystems 800, welche z.B. aus verschiedenen Betrachtungswinkeln mittels der Infrarot-Kameraeinheit erfasst wurden, kann insbesondere die optische Gestalt des Referenzsystems 800 oder der Marker berechnet werden, welche mit den ermittelten Informationen über die geometrische Gestalt des Referenzsystems 800 kombiniert werden kann. Die Korrelation von optischer und geometrischer Gestalt kann genutzt werden, um bei der Navigation, in Abhängigkeit vom Betrachtungswinkel des Referenzsystems durch die Kamera des Navigationssystems auftauchende Fehler aufgrund falscher Berechnung der räumlichen Lage durch z.B. beschädigte Marker zu kompensieren.

Die erfassten Informationen über das Instrument 700 und das Referenzsystem 800, das während des Scanvorganges vorzugsweise an dem Instrument 700 angeordnet ist, und die Position oder Qualität der Marker kann von der Scanvorrichtung 100 an eine Datenverarbeitungseinheit 400 übertragen werden, in welcher die erfassten Scandaten in ein virtuelles Modell umgewandelt werden. Die Datenverarbeitungseinheit 400 kann Funktionselemente oder Funktionseinheiten des Instruments 700 zum Beispiel direkt aus den erfassten Daten über die Geometrie des Instruments 700 und des Referenzsystems 800 erkennen oder ermitteln oder kann zum Beispiel unter Berücksichtigung der Geometrieverhältnisse des Instruments 700, wie der charakteristischen Anordnung der das Referenzsystem 800 bildenden Marker die zugehörigen Funktionselemente ermitteln, indem zum Beispiel die ermittelten Geometrieverhältnisse mit den in einer Datenbank gespeicherten Geometrieverhältnissen verglichen werden. Die Datenbank, in der die Vergleichswerte oder Vergleichsgeometrieverhältnisse gespeichert sein können oder neu ermittelte Geometrieverhältnisse gespeichert werden können, kann insbesondere in der Datenverarbeitungseinheit 400 angeordnet sein. Das von der Datenverarbeitungseinheit 400 errechnete virtuelle dreidimensionale Modell des Instruments 700 oder die ermittelten Informationen über die Geometrie des Instruments 700 und des Referenzsystems 800 können auf einer Anzeigevorrichtung 300, wie einem Touchscreen, angezeigt oder grafisch dargestellt werden. Insbesondere können auch verschiedene Alternativformen oder -geometrien des Instruments 700 oder der Funktionselemente des Instruments 700 angezeigt werden, deren Form oder Geometrie ähnlich den ermittelten Geometrien oder Funktionseinheiten des Instruments sind, woraus ein Benutzer ein Instrument 700 oder ein Funktionselement auswählen kann. Die ermittelten Informationen, wie die Geometrieverhältnisse des Instruments 700 und des Referenzsystems 800 oder das virtuelle Modell des Instruments 700 können von der Datenverarbeitungseinheit 400 drahtgebunden oder drahtlos, wie mittels WLAN oder Bluetooth, über eine Kommunikationseinheit 500, die an der Datenverarbeitungseinheit 400 und dem Navigationssystem 600 angeordnet sein kann, an das Navigationssystem übertragen werden, so dass anhand der ermittelten Informationen über die Geometrie des Instruments 700 ein Navigationsvorgang von dem Navigationssystem 600 durchgeführt werden kann. Bei dem Navigationsvorgang können die ermittelten Informationen über die Geometrie des Instruments 700 und des Referenzsystems 800 berücksichtigt werden und es können auch Informationen über den Zustand oder die Qualität der Marker berücksichtigt werden, so dass zum Beispiel eine Beschädigung der Marker oder Veränderungen der Geometrie oder Beschädigungen des Instruments bei dem Navigationsvorgang berücksichtigt werden können, um ein genauen Navigationsvorgang zu gewährleisten. Die Zeiterfassungseinheit 880 erfasst die Zeit der Messung. Die erfasste Zeit wird entweder direkt an das RFID-Lese-/Schreibgerät übermittelt, das dann die Zeitdaten in den RFID-Tag schreibt oder die Zeiterfassungseinheit gibt zuerst Zeitdaten an die Datenverarbeitungseinheit 400, die diese dann an das RFID-Lese-/Schreibgerät übermittelt. Alternativ und bevorzugt zusätzlich können die Zeitdaten an das Navigationssystem 600 übermittelt werden.

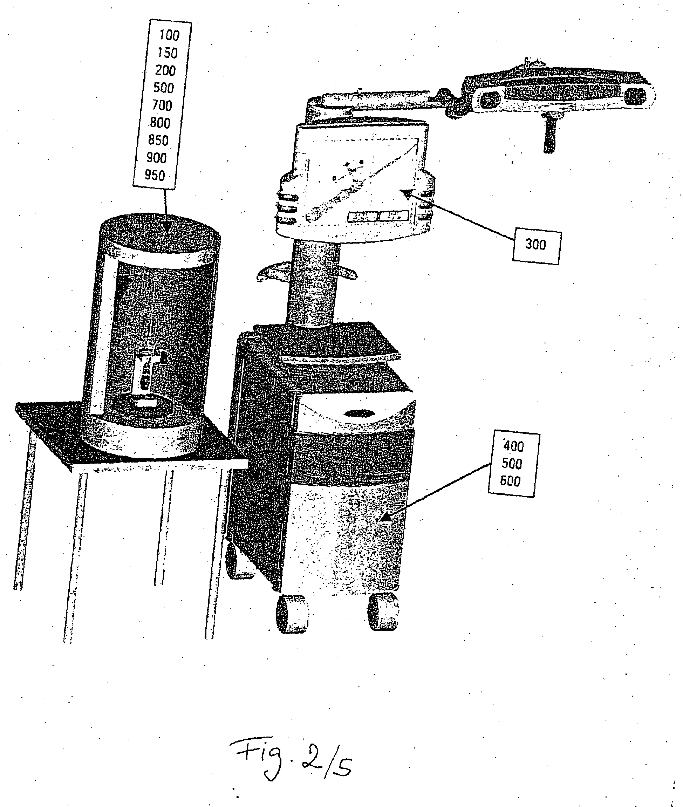

Figur 2 zeigt eine weitere Ausführungsform der vorliegenden Erfindung, wobei die Datenverarbeitungseinheit 400 in dem Navigationssystem 600 integriert ist bzw. in dem Navigationssystem angeordnet ist und die Anzeigevorrichtung 300 in das Navigationssystem 600 integriert ist bzw. an dem Navigationssystem 600 angeordnet ist.

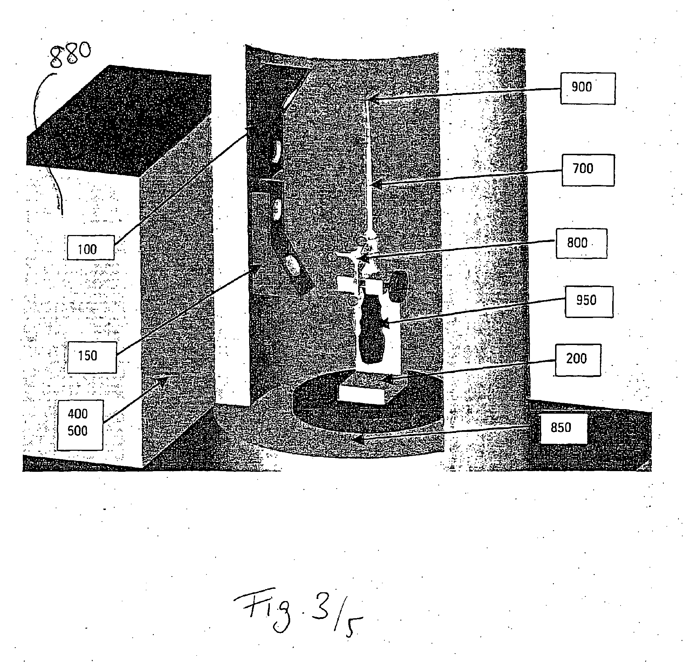

Figur 3 zeigt ein Gehäuse der Scanvorrichtung, in welchem das Instrument 700 positioniert ist, mit einer Scaneinheit 100, einer Infrarot-Kameraeinheit 150 und einem Instrumentenhalter 200, in welchem das Instrument 700 vorzugsweise fest positioniert ist. Das Instrument 700 besitzt als Funktionselement eine Instrumentenspitze 900, deren Form zum Beispiel von der Scaneinheit 100 erfasst werden kann. Auch ist an dem Instrument 700 ein Referenzsystem 800 angeordnet, dessen Form zum Beispiel von der Scaneinheit 100 erfasst werden kann, wobei das Referenzsystem 800 durch Marker gebildet wird, deren Reflexionscharakteristik zum Beispiel von der Infrarot-Kameraeinheit 150 erfasst werden kann. Bei dem gezeigten Beispiel befindet sich der RFID-Tag 950 im Handgriff des Instruments. Die im RFID-Tag gespeicherten Daten werden durch das RFID-Lesegerät 850 gelesen.

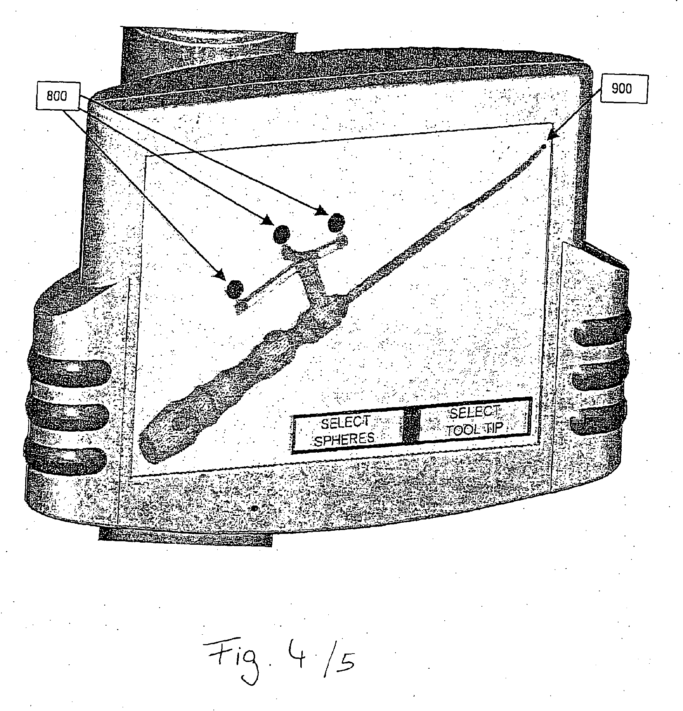

In Figur 4 ist die Anzeigevorrichtung 300 der vorliegenden Erfindung dargestellt, welche das virtuelle ermittelte dreidimensionale Modell des Instruments mitsamt Referenzsystem grafisch darstellt. Mittels einer Benutzerschnittstelleneinheit 1000, die vorzugsweise an der Anzeigevorrichtung 300 angeordnet ist, können zum Beispiel die Marker von einem Benutzer ausgewählt werden oder es können Funktionselemente, wie die Spitze 900 des Instruments, von einem Benutzer ausgewählt werden.

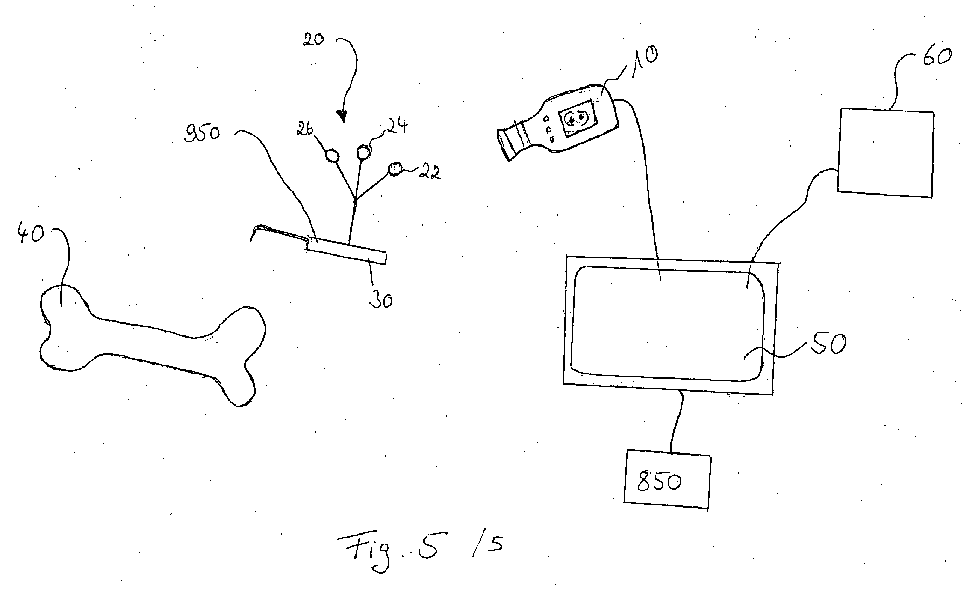

Figur 5 zeigt ein erfindungsgemäßes Navigationsgerät (Navigationssystem) mit einer Kamera 10 zum Detektieren einer Markereinrichtung 20 mit Markerkugeln 20, 24, 26. Die Markereinrichtung 20 ist bevorzugt als Referenzstern ausgebildet. Der Referenzstern 20 ist an einem Instrument 30 zum Behandeln einer Körperstruktur 40, z.B. Knochen eines Menschen angebracht. Der Referenzstern 20 bildet zusammen mit dem Instrument 30 (z.B. Messer) eine medizintechnische Behandlungsvorrichtung. Die Markerkugeln können passiv oder aktiv sein und werden von einer Kamera, die ein Beispiel für eine Detektionseinrichtung darstellt, detektiert. Die Detektionssignal von der Kamera 10 werden an eine Datenverarbeitungseinrichtung 50 übermitteln.

In dem Instrument 30 befindet sich ein RFID-Tag, der von einem RFID-Lesegerät 850 gelesen werden kann. Das Lesegerät 850 ist mit der Datenverarbeitungseinheit 50 gekoppelt, um die gelesenen Daten an die Datenverarbeitungseinrichtung 50 zu übermitteln. Die gelesenen Daten umfassen vorzugsweise Daten über die geometrischen Eigenschaften des Instruments 30 und/oder des Referenzsterns 20. Insbesondere kann der RFID-Tag 950 mit dem Instrument 30 oder mit dem Referenzstern 20 verbunden sein, z.B. daran angeklebt oder in seinem Inneren befindlich sein.

Bei den ausgelesenen Daten handelt es sich bevorzugt um die geometrischen Eigenschaften des Instruments 30 und/oder des Referenzsterns 20. Weiter sind Zeitdaten enthalten, die den Zeitpunkt der Messung der geometrischen Eigenschaften darstellen. Gemäß einer zusätzlichen Ausführungsform sind weiter Daten enthalten, die den Typ des Instruments und/oder des Referenzsterns beschreiben. Alle diese Daten werden bevorzugt von dem Lesegerät 850 gelesen und zu der Datenverarbeitungseinrichtung 50 übermittelt.

Die Datenverarbeitungseinrichtung 50 errechnet aus dem Zeitpunkt der Messung einen Soll-Zeitpunkt, bis zu dem eine Gültigkeit der Daten für die geometrischen Eigenschaften gegeben ist. Diese Soll-Zeit kann auch als Verfallsdatum bezeichnet werden.

Gemäß einer alternativen Ausführungsform der Erfindung wird alternativ oder zusätzlich zu der Zeit der Messung in den RFID-Transponder der medizintechnischen Behandlungsvorrichtung die vorgenannte Soll-Zeit gespeichert. D.h. die Datenverarbeitungseinrichtung, die bei der Messung der geometrischen Eigenschaften der medizintechnischen Behandlungsvorrichtung verwendet wird, berechnet aus der gemessenen Zeit, bei Messung der geometrischen Daten mittels der Messvorrichtung die vorgenannte Soll-Zeit, die dann in den RFID-Transponder der medizintechnischen Behandlungsvorrichtung mittels des RFID-Schreibgeräts geschrieben wird.

Ist diese Soll-Zeit, die auch als Verfallszeit bezeichnet werden kann, in den RFID-Transponder eingespeichert, so kann diese Soll-Zeit von dem Lesegerät 850, das in Figur 5 gezeigt ist, ausgelesen werden.

In der Datenverarbeitungseinrichtung 50 wird als somit eine Soll-Zeit errechnet und/oder wird von dem Lesegerät 850 empfangen. Diese Soll-Zeit wird bevorzugt mit der aktuellen Zeit verglichen. Liegt die Soll-Zeit vor der aktuellen Zeit, so wird bevorzugt eine Soll-Zeit-Überschreitungsoperation durchgeführt. Hierbei wird beispielsweise ein Warnsignal in der Anzeigeeinrichtung 60 dargestellt. Die Anzeigeeinrichtung 60 kann zum Darstellen der Position des Instruments 30 dienen. Alternativ oder zusätzlich kann die Darstellung des Instruments 30 auf dem Anzeigegerät 60 unterbunden werden, indem beispielsweise das Anzeigegerät 60 durch Steuerung der Datenverarbeitungseinrichtung 50 ausgeschaltet wird. Als Warnsignal kann natürlich alternativ oder zusätzlich ein Warntonsignal abgegeben werden.

Wie oben ausgeführt, kann gemäß einer alternativen Ausführungsform auch ein Identifikationscode aus dem RFID-Transponder 59 ausgelesen werden, der die medizintechnische Behandlungsvorrichtung spezifiziert. Berechnet die Datenverarbeitungseinheit 50 die Soll-Zeit, so kann die Berechnung dieser Soll-Zeit in Abhängigkeit von dem identifizierten Instrument vorgenommen werden. Dazu greift die Datenverarbeitungseinrichtung 50 beispielsweise auf eine Datenbank zu, die für verschieden Instrumententypen unterschiedliche Gültigkeitszeitdauern ab Messzeitpunkt der geometrischen Eigenschaften festlegt. Dies ist sinnvoll, da verschiedene medizintechnische Behandlungsvorrichtungen unterschiedliche mechanische Stabilität aufweisen und einer unterschiedlichen typischen Belastung im medizinischen Alltag unterworfen sind. Vorzugsweise greift also die Datenverarbeitungseinrichtung 50 in Abhängigkeit von dem Identifikationscode auf beispielsweise eine Tabelle zu, bei der festgelegt wird, welcher Instrumententyp zu dem Identifikationscode des Instruments passt. In Abhängigkeit von dem Typ wird dann aus einer Tabelle die Soll-Zeitdauer Abmessung der geometrischen Eigenschaften ausgelesen. Mittels der Soll-Zeitdauer und der ausgelesenen Zeit der Messung der geometrischen Eigenschaften wird dann die vorgenannte Soll-Zeit (Verfallszeit) berechnet. Alternativ oder zusätzlich kann zu dem Code, der die einzelne medizintechnische Behandlungsvorrichtung charakterisiert, auch ein Code aus dem RFID-Transponder ausgelesen werden, der den Typ des Instruments charakterisiert, so dass innerhalb der Datenverarbeitungseinrichtung 50 nicht auf eine Tabelle zurückgegriffen werden muss, die eine Verbindung zwischen dem Identifikationscode und dem Typ des Instruments herstellt.

Die vorgenannt beschriebenen Eigenschaften der Datenverarbeitungseinrichtung 50 können auch in der Datenverarbeitungseinrichtung 400 realisiert sein, die bei der Messung der geometrischen Eigenschaften der medizintechnischen Behandlungsvorrichtung eingesetzt wird. Auf diese Art und Weise kann bereits bei der Messung der Eigenschaften der medizintechnischen Behandlungsvorrichtung eine Soll-Zeit bestimmt werden und dies alternativ oder zusätzlich zu der Zeit der Messung von der erfindungsgemäßen Messvorrichtung mittels des RFID-Schreibgeräts in den RFID-Transponder der medizintechnischen Behandlungsvorrichtung eingeschrieben werden. Insbesondere kann auch der Typ der medizintechnischen Behandlungsvorrichtung in den RFID-Transponder geschrieben werden.

## Patentansprüche

1. Messvorrichtung zum Messen geometrischer Eigenschaften einer medizintechnischen Behandlungsvorrichtung (700, 800) mit einer Messeinheit (100), welche ausgebildet ist, die geometrische Eigenschaft zu messen, wobei die geometrische Eigenschaft die relative Lage zwischen mindestens einem ersten Teil der medizintechnischen Behandlungsvorrichtung (700, 800) und mindestens einem zweiten Teil der medizintechnischen Behandlungsvorrichtung (700, 800) ist; **gekennzeichnet durch**
- eine Zeiterfassungseinheit (880), die ausgebildet ist, die Zeit der Messung zu erfassen;
- ein RFID-Schreibgerät, das ausgebildet ist, eine Zeit, die auf der erfassten Zeit basiert, in einen RFID-Transponder der medizintechnischen Behandlungsvorrichtung zu schreiben, wobei
- das RFID-Schreibgerät dazu eingerichtet ist, die gemessene geometrische Eigenschaft in den RFID-Transponder zu schreiben oder die Messvorrichtung Mittel aufweist, um die gemessene geometrische Eigenschaft in eine Datenbank zu speichern,

2. Messvorrichtung nach Anspruch 1, bei der die Zeit, die auf der erfassten Zeit basiert, die erfasste Zeit ist, oder eine Zeit ist, die aus der erfassten Zeit mittels einer Datenverarbeitungseinrichtung (400) berechnet wird.

3. Messvorrichtung nach Anspruch 2, bei der die berechnete Zeit basierend auf einem Soll-Zeitraum und der erfassten Zeit berechnet wird, um so eine Soll-Zeit zu berechnen, bis zu der die geometrischen Eigenschaften der medizintechnischen Behandlungsvorrichtung (700, 800) als gültig angesehen werden.

4. Messvorrichtung nach Anspruch 3, bei der die Datenverarbeitungseinrichtung (400) auf eine Datenbank zugreift, in der unterschiedliche Soll-Zeiträume verschiedenen Typen von medizintechnischen Behandlungsvorrichtung (700, 800) zugeordnet sind, und die medizintechnischen Behandlungsvorrichtung (700, 800) in Abhängigkeit vom Typ der gemessenen medizintechnischen Behandlungsvorrichtung (700, 800) mit Hilfe der Datenbank den zum Typ gehörigen Soll-Zeitraum bestimmt und in Abhängigkeit vom bestimmten Soll-Zeitraum die Soll-Zeit berechnet.

5. Messvorrichtung nach einem der vorhergehenden Ansprüche, bei dem die Datenverarbeitungseinrichtung (400) in Abhängigkeit von den gemessenen geometrischen Eigenschaften der medizintechnischen Behandlungsvorrichtung (700, 800) den Typ der medizintechnischen Behandlungsvorrichtung (700, 800) bestimmt.

6. Navigationssystem (600) zum Navigieren einer medizintechnischen Behandlungsvorrichtung (30) insbesondere zur medizinischen Behandlung von Körperstrukturen, insbesondere bei Operationen, wobei das Navigationssystem (600) umfasst:
eine Detektionseinrichtung (10) zum Detektieren eines Referenzsystems, das die medizintechnische Behandlungsvorrichtung (30) umfasst;
eine Datenverarbeitungseinrichtung (50), um die gemessenen Daten zu verarbeiten; **gekennzeichnet durch**
ein RFID-Lesegerät, um aus einem RFID-Transponder der medizintechnischen Behandlungsvorrichtung (30) eine Zeit, die auf der Zeit einer Bestimmung einer geometrischen Eigenschaft der medizinischen Behandlungsvorrichtung basiert, auszulesen;
wobei die geometrische Eigenschaft die relative Lage zwischen mindestens einem ersten Teil der medizinischen Behandlungsvorrichtung (30) und mindestens einem zweiten Teil der medizinischen Behandlungsvorrichtung (30) ist,
wobei die Datenverarbeitungseinrichtung (50) ausgebildet ist, aus der ausgelesenen Zeit eine Soll-Zeit zu bestimmen, bis zu der eine Gültigkeit der geometrischen Eigenschaft angenommen wird und
wobei das RFID-Lesegerät dazu eingerichtet ist, die geometrische Eigenschaft aus dem RFID-Transponder auszulesen oder das Navigationssystem dazu eingerichtet ist, die geometrische Eigenschaft aus einer Datenbank auszulesen.

7. Navigationssystem (600) nach Anspruch 6, bei welchem das Navigationssystem (600) eine Soll-Zeit-Überschreitungsoperation durchführt, wenn die aktuelle Zeit nach der Soll-Zeit liegt.

8. Navigationssystem (600) nach einem der Ansprüche 6 oder 7, bei welchem die Soll-Zeit-Überscheitungsoperation die Abgabe eines Warnsignals und/oder das Unterbinden einer Anzeige von zur Navigation notwendigen Daten und/oder ein Sperren des Navigationssystems (600) für den Benutzer umfasst.

9. Medizintechnische Behandlungsvorrichtung (700, 30) mit einem RFID-Transponder, in dem eine Zeit, die auf der Zeit einer Bestimmung einer geometrischen Eigenschaft der medizintechnischen Behandlungsvorrichtung (700, 30) basiert, enthalten ist, und einem Referenzsystem, wobei die geometrische Eigenschaft die relative Lage zwischen mindestens einem ersten Teil der medizinischen Behandlungsvorrichtung (30) und mindestens einem zweiten Teil der medizinischen Behandlungsvorrichtung (30) ist,
wobei in dem RFID-Transponder die geometrische Eigenschaft der medizintechnischen Behandlungsvorrichtung (700, 30) enthalten ist.

10. Verfahren zum Messen geometrischer Eigenschaften einer medizintechnischen Behandlungsvorrichtung (700, 30), bei welchem die geometrische Eigenschaft gemessen wird, die die relative Lage zwischen einem ersten Teil der medizintechnischen Behandlungsvorrichtung (700, 30) und einem zweiten Teil der medizintechnischen Behandlungsvorrichtung (700, 30) ist; **dadurch gekennzeichnet, dass**
die Zeit der Messung erfasst wird;
eine Zeit, die auf der erfassten Zeit basiert, in einen RFID-Transponder der medizintechnischen Behandlungsvorrichtung (700, 30) geschrieben wird und
die geometrische Eigenschaft in den RFID-Transponder oder in eine Datenbank geschrieben wird.

11. Computerprogramm, das, wenn es in eine Messvorrichtung nach einem der Ansprüche 1 bis 5 geladen wird oder wenn es auf einer solchen Messvorrichtung läuft, die Messvorrichtung dazu veranlasst, dass Verfahren nach Anspruch 10 durchzuführen.

## Claims

1. Measuring device for measuring geometric properties of a medical treatment device (700, 800) with a measuring unit (100) configured to measure a geometric property, which geometric property is the relative position between at least a first part of the medical treatment device (700, 800) and at least a second part of the medical treatment device (700, 800), **characterised by**
- a time detection unit (880) configured to detect the time of the measurement;
- an RFID writing device configured to write a time based on the detected time to an RFID transponder of the medical treatment device, and
- the RFID writing device is configured to write the measured geometric property to the RFID transponder or the measuring device has means for storing the measured geometric property in a data bank.

2. Measuring device as claimed in claim 1, whereby the time based on the detected time is the detected time or a time calculated from the detected time by means of a data processing unit (400).

3. Measuring device as claimed in claim 2, whereby the calculated time is calculated on the basis of a desired period of time and the detected time in order to calculate a desired time up to which the geometric properties of the medical treatment device (700, 800) are regarded as valid.

4. Measuring device as claimed in claim 3, whereby the data processing unit (400) accesses a data bank in which various desired periods of time are assigned to different types of medical treatment devices (700, 800) and, based on the type of medical treatment device (700, 800) measured, determines the desired period of time for the medical treatment device (700, 800) with the aid of the data bank, based on the associated type, and calculates the desired time on the basis of the retrieved desired period of time.

5. Measuring device as claimed in one of the preceding claims, whereby the data processing unit (400) determines the type of medical treatment device (700, 800) on the basis of the measured geometric properties of the medical treatment device (700, 800).

6. Navigation system (600) for navigating a medical treatment device (30), in particular for the medical treatment of body structures, in particular during operations, which navigation system (600) comprises:
a detection unit (10) for detecting a reference system containing the medical treatment device (30);
a data processing unit (50) for processing the measured data;
**characterised by**
an RFID reading device for reading from an RFID transponder of the medical treatment device (30) a time based on the time taken to detect a geometric property of the medical treatment device;
and the geometric property is the relative position between at least a first part of the medical treatment device (30) and at least a second part of the medical treatment device (30),
and the data processing unit (50) is configured to determine from the read time a desired period of time up to which the geometric property is accepted as being valid and
the RFID reading device is configured to read the geometric property from the RFID transponder or the navigation system is configured to read the geometric property from a data bank.

7. Navigation system (600) as claimed in claim 6, which navigation system (600) runs a desired time overrun operation if the current time lies after the desired time.

8. Navigation system (800) as claimed in one of claims 6 or 7, whereby the desired time overrun operation involves emitting a warning signal and/or preventing a display of data needed for navigation purposes and/or denying the user access to the navigation system (600).

9. Medical treatment device (700, 30) with an RFID transponder containing a time based on the time taken to determine a geometric property of the medical treatment device (700, 30), and a reference system in which the geometric property is the relative position between at least a first part of the medical treatment device (30) and at least a second part of the medical treatment device (30),
and the geometric property of the medical treatment device (700, 30) is contained in the RFID transponder.

10. Method of measuring geometric properties of a medical treatment device (700, 30), whereby the geometric property, which is the relative position between a first part of the medical treatment device (700, 30) and a second part of the medical treatment device (700, 30), is measured;
**characterised in that**
the time of the measurement is detected;
a time based on the detected time is written to an RFID transponder of the medical treatment device (700, 30) and
the geometric property is written to the RFID transponder or to a data bank.

11. Computer programme which prompts the measuring device to run the method as claimed in claim 10 when loaded onto a measuring device as claimed in one of claims 1 to 5 or when running on such a measuring device.

## Revendications

1. Dispositif de mesure pour mesurer des propriétés géométriques d'un dispositif de traitement médico-technique (700, 800) à l'aide d'une unité de mesure (100) qui est configurée pour mesurer la propriété géométrique, dans lequel la propriété géométrique est la position relative entre au moins une première partie du dispositif de traitement médico-technique (700, 800) et au moins une seconde partie du dispositif de traitement médico-technique (700, 800), **caractérisé par**
- une unité de détection temporelle (880) configurée pour détecter une durée de la mesure,
- un dispositif d'écriture RFID configuré pour écrire un temps basé sur la durée détectée dans un transpondeur RFID du dispositif de traitement médico-technique, dans lequel
- le dispositif d'écriture RFID est conçu de manière à écrire la propriété géométrique mesurée dans le transpondeur RFID, ou le dispositif de mesure comporte des moyens pour enregistrer la propriété géométrique mesurée dans une base de données.

2. Dispositif de mesure selon la revendication 1, dans lequel le temps basé sur la durée détectée est la durée détectée ou est un temps calculé à partir de la durée détectée au moyen d'un dispositif de traitement de données (400).

3. Dispositif de mesure selon la revendication 2, dans lequel le temps calculé est calculé sur la base d'un intervalle de temps de consigne et de la durée détectée, de manière à calculer un temps de consigne jusqu'à ce que les propriétés géométriques du dispositif de traitement médico-technique (700, 800) soient considérées comme valides.

4. Dispositif de mesure selon la revendication 3, dans lequel le dispositif de traitement de données (400) accède à une base de données dans laquelle différents intervalles de temps de consigne sont affectés à différents types de dispositif de traitement médico-technique (700, 800), et le dispositif de traitement médico-technique (700, 800) détermine l'intervalle de temps de consigne associé au type à l'aide de la base de données en fonction du type du dispositif de traitement médico-technique mesuré (700, 800) et calcule le temps de consigne en fonction de l'intervalle de temps de consigne déterminé.

5. Dispositif de mesure selon l'une quelconque des revendications précédentes, dans lequel le dispositif de traitement de données (400) détermine le type du dispositif de traitement médico-technique (700, 800) en fonction des propriétés géométriques mesurées du dispositif de traitement médico-technique (700, 800).

6. Système de navigation (600) pour la navigation d'un dispositif de traitement médico-technique (30), en particulier pour le traitement médical de structures corporelles, en particulier lors d'opérations, le système de navigation (600) comportant :
un dispositif de détection (10) pour détecter un système de référence incluant le dispositif de traitements médico-technique (30),
un dispositif de traitement de données (50) pour traiter les données mesurées, **caractérisé par**
un dispositif de lecture RFID pour lire à partir d'un transpondeur RFID du dispositif de traitement médico-technique (30) un temps basé sur le temps d'une détermination d'une propriété géométrique du dispositif de traitement médico-technique,
dans lequel la propriété géométrique est la position relative entre au moins une première partie du dispositif de traitement médical (30) et au moins une seconde partie du dispositif de traitement médical (30),
dans lequel le dispositif de traitement de données (50) est configuré pour déterminer, à partir du temps lu, un temps de consigne jusqu'à ce que la propriété géométrique soit supposée valide et
dans lequel le dispositif de lecture RFID est configuré pour lire la propriété géométrique à partir du transpondeur RFID, ou le système de navigation est configuré pour lire la propriété géométrique à partir d'une base de données.

7. Système de navigation (600) selon la revendication 6, dans lequel le système de navigation (600) exécute une opération de dépassement de temps de consigne lorsque le temps actuel est au-delà du temps de consigne.

8. Système de navigation (600) selon l'une quelconque des revendications 6 ou 7, dans lequel l'opération de dépassement de temps de consigne comporte les étapes consistant à émettre un signal d'avertissement et/ou empêcher un affichage de données nécessaires pour la navigation et/ou empêcher l'utilisation du système de navigation (600) par l'utilisateur.

9. Dispositif de traitement médico-technique (700, 30) muni d'un transpondeur RFID contenant un temps basé sur la durée d'une détermination d'une propriété géométrique du dispositif de traitement médico-technique (700, 30), et d'un système de référence, dans lequel la propriété géométrique est la position relative entre au moins une première partie du dispositif de traitement médical (30) et au moins une seconde partie du dispositif de traitement médical (30),
dans lequel le transpondeur RFID contient la propriété géométrique du dispositif de traitement médico-technique (700, 30).

10. Procédé pour mesurer des propriétés géométriques d'un dispositif de traitement médico-technique (700, 30), comportant l'étape consistant à mesurer la propriété géométrique, ladite propriété étant la position relative entre une première partie du dispositif de traitement médico-technique (700, 30) et une seconde partie du dispositif de traitement médico-technique (700, 30), **caractérisé par** les étapes consistant à
détecter la durée de la mesure,
écrire un temps basé sur la durée détectée dans un transpondeur RFID du dispositif de traitement médico-technique (700, 30) et
écrire la propriété géométrique dans le transpondeur RAID ou dans une base de données.

11. Programme informatique qui, lorsqu'il est chargé dans un dispositif de mesure selon l'une quelconque des revendications 1 à 5 ou lorsqu'il s'exécute sur un tel dispositif de mesure amène le dispositif de mesure à exécuter le procédé selon la revendication 10.
